Europäisches Patentamt

European Patent Office

Office européen des brevets

(19)

(11) Publication number: **0 308 269 B1**

(12) **EUROPEAN PATENT SPECIFICATION**

(45) Date of publication of patent specification: **23.12.92**   (51) Int. Cl.⁵: **A61M 36/00**

(21) Application number: **88308636.5**

(22) Date of filing: **19.09.88**

(54) **Cartridge for pellets.**

(30) Priority: **18.09.87 AU 4456/87**
**21.09.87 AU 4473/87**
**19.01.88 AU 6354/88**

(43) Date of publication of application:
**22.03.89 Bulletin 89/12**

(45) Publication of the grant of the patent:
**23.12.92 Bulletin 92/52**

(84) Designated Contracting States:
**AT BE CH DE ES FR GB GR IT LI LU NL SE**

(56) References cited:
**EP-A- 0 090 899**
**GB-A- 840 276**
**GB-A- 1 583 816**
**US-A- 4 531 938**

(73) Proprietor: **Hoechst Veterinär GmbH**
**Feldstrasse 1a**
**W-8044 Unterschleissheim(DE)**

(72) Inventor: **Ball, Keith Vernon**
**36 Liston Street**
**Burwood Victoria(AU)**

(74) Representative: **Schmidt, Werner, Dr. et al**
**Hoechst AG Zentrale Patentabteilung Post-**
**fach 80 03 20**
**W-6230 Frankfurt am Main 80(DE)**

Rank Xerox (UK) Business Services

## Description

This invention relates to an improved cartridge for transporting pellets for use in an implant gun or the like, and to a supply of pellets provided in such cartridge for a gun.

In a variety of guns, such as the implant guns disclosed in European Patent Specification No 0309172, there is a need for a device in which pellets or the like are transportable in turn to a discharge position.

It is convenient to locate the pellets in a cartridge belt, which may be transported between separate discharges of pellets to bring the next pellet to the discharge position. Typically, cartridge belts are provided with the holders for the pellets disposed with their axes parallel to the face of a flat belt on which or in which the holders are mounted, for example as is disclosed in US Patent No 4531938.

The present invention provides a cartridge body or belt in which the holders for the pellets are disposed with their axes substantially perpendicular to the face of the belt.

Accordingly, the present invention provides a cartridge for use in transporting pellets for use in an implant gun or the like, the cartridge comprising an elongate cartridge body having an elongate carrier strip and a plurality of hollow holders for respective pellets substantially uniformly spaced along said carrier strip, each holder defining a respective cavity such that each holder is able to hold at least one pellet, the carrier strip being of ribbon form, characterized in that the ribbon which forms the carrier strip is flat and the holders are disposed along a major face of the carrier strip and the axis of each holder along which the pellet is intended to be discharged is substantially perpendicular to said major face of the carrier strip.

The cartridge body preferably is flexible, such as by being formed from a suitable plastics material. Low density polyethylene or a similar grade of polypropylene are particularly suitable. However, other plastics materials with similar physical properties can be used.

The holders preferably are centrally disposed on the major face of the carrier strip.

The cavities, and most preferably the holders externally, preferably are configured so as to conform substantially to at least one section of the pellets. The pellets most conveniently are of substantially circular cross-section in at least one plane, such as being of spherical or cylindrical form. The cavity of each holder, for such pellets, most preferably is of substantially cylindrical form.

Each holder has at least one opening communicating with its cavity, to enable at least one pellet to be inserted into or ejected from the cavity.

Preferably, each holder has two opposed openings, each communicating with its cavity; one opening enabling access of a pellet ejecting device, such as an ejecting pin, through which such device can extend to eject the pellet or pellets through the other opening.

Preferably each holder is hollow and has each end open; with access to the end thereof adjacent the strip being provided by a respective opening through the strip. In such form, each holder and its cavity may taper slightly away from the strip. This facilitates production of the cartridge, which most preferably is formed by injection moulding of a suitable thermoplastics material. For the same reason, but also to facilitate insertion of pellets into the holders, the openings in the strip may be of counter-sunk form at the major face of the strip remote from the holders. Also the opening of the holders remote from the strip may be partially obstructed, such as by provision of a marginal flange. Alternatively, a number of small protuberances can be formed around that opening. The holders, after at least one pellet is provided in each, may be at least partially closed. Thus, where the holders are of the above-described slightly tapered form, a thin film or foil may be applied over the remote face of the strip to retain the pellets therein. Such film or foil most preferably is readily able to be perforated, and may be heat sealed or adhesively bonded to the strip. Alternatively, an opening to each cavity adjacent the strip may be partially closed by a marginal flange or protruberances past which the pellets are forced into each cavity; the flange or protrubances being able to recover resiliently to retain the pellets in the cavity.

In order that the invention may more readily be understood, reference now is directed to the accompanying drawings, in which:

Figures 1 and 2 show, in side elevation and top plan view, cartridges of typical approximate actual size, as formed by injection moulding for use with an implant gun as described in our above-described co-pending Australian patent application PI 4456;

Figure 3 is a view, in side elevation, of part of the cartridge of Figure 1, on a scale of about 10:1;

Figure 4 is a bottom plan view of the part cartridge of Figure 3; and

Figure 5 is sectional view, taken on line V-V of Figure 3.

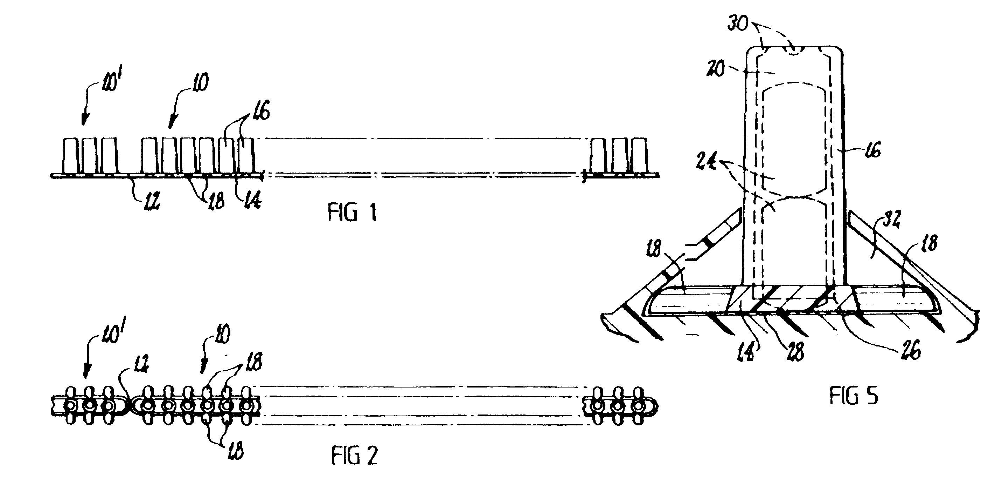

The cartridge 10 of Figures 1 and 2 is formed by injection moulding low density polyethylene. Typically an end to end connected series of cartridges are formed simultaneously, part of a second cartridge 10' being shown in each of Figures 1 and 2. After moulding, the cartridges readily are able to be separated by rupturing the thin bridging elements 12 therebetween.

Cartridge 10 has a thin basal strip 14, from one face of which projects a series, typically of about 20 or 25, of pellet holders 16. To each side of each holder 16, strip 14 has a respective laterally projecting guide tab 18.

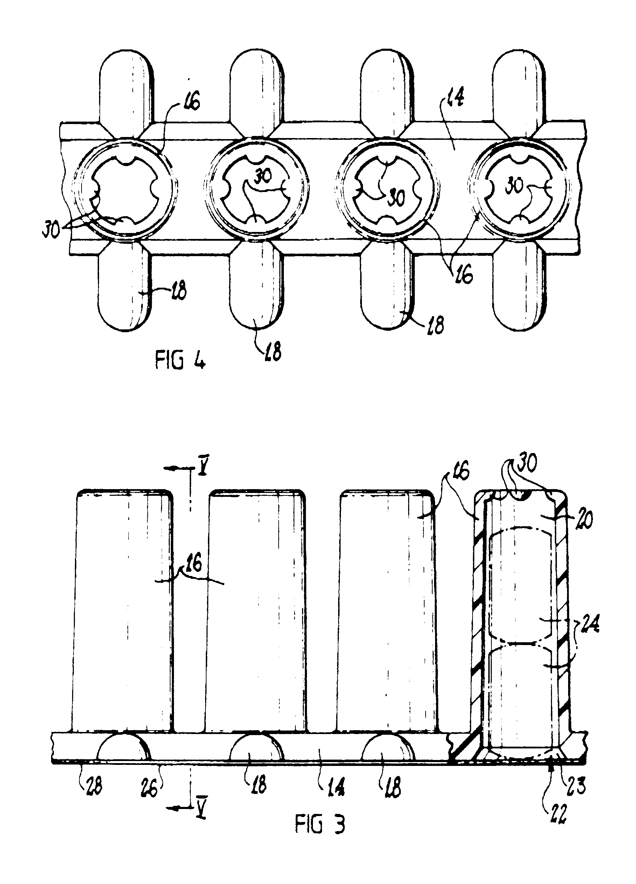

As shown in Figure 3; holders 16 are of hollow, substantially cylindrical form. However, each tapers slightly away from strip 14.

The cavity 20 of each holder 16 communicates with a respective opening 22 in strip 14; with each opening having a flared inlet 23 thereto.

Cartridge 10 is intended to hold two cylindrical pellets 24 in each holder 16. Such pellets 24 are shown in broken outline at the time of insertion into the holder 16 shown in section in Figure 3. After the pellets 24 are inserted into each holder 16, openings 22 are closed by application of a thin, perforatable layer 26 of plastics film, metal foil, or a laminate material over bottom surface 28 of strip 14. Layer 26 is shown as if applied to surface 28 but, obviously, it is applied, such as by heat sealing or adhesive bonding, after insertion of the required pellets 24.

The end of holders 16 remote from strip 14 is configured to retain pellets 24 therein. For this purpose, a number of radially inwardly projecting protruberances 30 are provided around cavity 20. The radial extent of protruberances 30 is such that they prevent inadvertent passage of pellets 24, but enable the latter to be ejected by a moderate force. This arrangement is sufficient where the pellets do not require protection from air and/or moisture. However, where such protection is required, a thin membrane can be provided across that remote end of cavity 20. Such membrane can be additional to, or instead of, protruberances 30. The membrane can be produced as part of cartridge 10 by injection moulding. Alternatively, the membrane may comprise a thin film or foil applied across the remote end of holders 16, such as by heat sealing in adhesive bonding.

In use of cartridge 10 in an implant gun of our above-described co-pending application, it is inserted end-wise into a guideway 32 of a pellet discharge device, such as an implant gun as disclosed in our above-mentioned Australian patent application PI 4456. As shown, guideway 32 has a form somewhat complementary to that of the cartridge as shown in Figure 5. The cartridge is then indexed along the guideway 32, to present each holder and its pellet(s) in turn to a discharge position of such device. Guide tabs 18 enagle such indexing under the action of an indexing means, such as a pair of ratchet wheels, engageable with tabs 18. An ejecting pin then is inserted through the opening 22 of a holder at such position, perforating the film or foil 26 of the holder, to eject the pellet(s) through protruberances 30. Tabs 18 assist in guiding cartridge 10 along guideway 32. However, they also facilitate bending of cartridge 10 around any curve in guideway 32 and, more importantly, they enable indexing means to engage cartridge 10 and to impart indexed, endwise movement to cartridge 10.

The pellets can be of a wide variety of forms. In one form, they are for implantation into or below animal tissue, and may comprise a variety of drugs, trace elements or like materials.

## Claims

1. A cartridge for use in transporting pellets for use in an implant gun or the like, the cartridge comprising an elongate cartridge body having an elongate carrier strip (14) and a plurality of hollow holders (16) for respective pellets substantially uniformly spaced along said carrier strip, each holder defining a respective cavity such that each holder is able to hold at least one pellet, the carrier strip (14) being of ribbon form, characterized in that the ribbon which forms the carrier strip (14) is flat and the holders (16) are disposed along a major face of the carrier strip and the axis of each holder along which the pellet is intended to be discharged is substantially perpendicular to said major face of the carrier strip.

2. A cartridge according to claim 1, wherein the cartridge body is flexible and is formed from a plastics material selected from low density polyethylene and polypropylene.

3. A cartridge according to any one of claim 1 or claim 2, wherein the cavity (20) of each holder (16) is configured so as to conform substantially to at least one cross-section of the pellets.

4. A cartridge according to claim 3, wherein the cavity (20) of each holder (16) is of substantially cylindrical form.

5. A cartridge according to any one of claims 1 to 4 wherein each holder (16) has at least one opening (22) communicating with its cavity (20) to enable at least one pellet to be inserted into or ejected from the cavity.

6. A cartridge according to claim 5, wherein each holder (16) has two opposed openings, each communicating with its cavity (20) one opening enabling access for a pellet ejecting device through which said device can extend to eject the at least one pellet through the other opening.

7.  A cartridge according to claim 5 or claim 6 wherein at least one opening is disposed in said strip (14) and is covered by a thin layer (26) of material readily able to be perforated to enable access therethrough to the cavity (20) of respective holders.

8.  A cartridge according to any one of claims 5 to 7 wherein each holder (16) has an opening at an end thereof remote from the strip, which opening is partially obstructed.

9.  A cartridge according to claim 8, wherein each remote opening is partially obstructed by provision of a marginal flange.

10. A cartridge according to claim 8, wherein each remote opening is partially obstructed by provision of a plurality of small protuberances (30) spaced around that opening.

11. A cartridge according to any one of claims 1 to 10, wherein a plurality of laterally projecting tabs (18) is provided along at least one side of said strip to provide means by which the cartridge body can be indexed along a guideway.

**Patentansprüche**

1.  Patrone für den Transport von Pellets zur Verwendung in einer Implantierpistole od.dgl., wobei die Patrone einen länglichen Patronenkörper mit einem länglichen Trägerstreifen (14) und einer Vielzahl von hohlen Haltern (16) für die jeweiligen Pellets in im wesentlichen gleichmäßigen Abständen entlang dieses Trägerstreifens aufweist, jeder Halter einen entsprechenden Hohlraum umfaßt, sodaß er zumindest ein Pellet aufnehmen kann, und der Trägerstreifen (14) bandförmig ist, dadurch gekennzeichnet, daß das den Trägerstreifen (14) bildende Band flach ist, daß die Halter (16) entlang einer Hauptfläche des Trägerstreifens angeordnet sind und daß die Achse jedes Halters, entlang welcher das Pellet abgegeben werden soll, im wesentlichen in einem rechten Winkel zu dieser Hauptfläche des Trägerstreifens steht.

2.  Patrone nach Anspruch 1, bei welcher der Patronenkörper biegbar und aus einem Kunststoffmaterial hergestellt ist, das aus Polyäthylen geringer Dichte und Polypropylen ausgewählt ist.

3.  Patrone nach Anspruch 1 oder 2, bei welcher der Hohlraum (20) jedes Halters (16) eine Form besitzt, die im wesentlichen zumindest einem Querschnitt der Pellets entspricht.

4.  Patrone nach Anspruch 3, bei welcher der Hohlraum (20) jedes Halters (16) im wesentlichen zylindrische Form aufweist.

5.  Patrone nach einem der Ansprüche 1 bis 4, bei welcher jeder Halter (16) zumindest eine Öffnung (22) aufweist, die mit seinem Hohlraum (20) in Verbindung steht, um zu ermöglichen, daß zumindest ein Pellet in den Hohlraum eingesetzt oder aus diesem ausgestoßen werden kann.

6.  Patrone nach Anspruch 5, bei welcher jeder Halter (16) zwei einander gegenüberliegende Öffnungen aufweist, die mit seinem Hohlraum (20) in Verbindung stehen, wobei eine Öffnung den Zugang der Pellet-Ausstoßvorrichtung ermöglicht und sich diese Vorrichtung durch die Öffnung erstrecken kann, um das zumindest eine Pellet durch die andere Öffnung auszustoßen.

7.  Patrone nach Anspruch 5 oder 6, bei welcher zumindest eine Öffnung in dem Streifen (14) vorgesehen und von einer dünnen Schicht (26) eines Materials bedeckt ist, das leicht perforiert werden kann, um einen Zugang durch dasselbe in den Hohlraum (20) des entsprechenden Halters zu ermöglichen.

8.  Patrone nach einem der Ansprüche 5 bis 7, bei welcher jeder Halter (16) an einem von dem Streifen abgewandten Ende eine Öffnung aufweist, die teilweise blockiert ist.

9.  Patrone nach Anspruch 8, bei welcher jede abgewandte Öffnung durch Anordnung eines Randflansches teilweise blockiert ist.

10. Patrone nach Anspruch 8, bei welcher jede abgewandte Öffnung durch Anordnung einer Vielzahl kleiner Vorsprünge (30), die rund um die Öffnung vorgesehen sind, teilweise blockiert ist.

11. Patrone nach einem der Ansprüche 1 bis 10, bei welcher eine Vielzahl seitlich abstehender Ansätze (18) entlang zumindest einer Seite des genannten Streifens vorgesehen ist, um eine Anordnung zu schaffen, mittels welcher der Patronenkörper entlang eines Führungsweges schrittweise weitergeführt werden kann.

**Revendications**

1. Cartouche servant à transporter des pellets ou comprimés à utiliser dans un pistolet à implanter ou analogue, comprenant un corps de cartouche de forme allongée possédant une bande (14) support de forme allongée et une pluralité de récipients (16) creux pour des comprimés respectifs, qui sont mutuellement espacés de façon sensiblement uniforme le long de la bande (14) support, chaque support définissant une cavité respective telle que chaque récipient peut contenir au moins un comprimé, la bande (14) support ayant la forme d'un ruban, caractérisée en ce que le ruban qui forme la bande (14) support est plat, les récipients (16) sont disposés le long d'une grande face de la bande (14) support et l'axe de chaque récipien, axe suivant lequel le comprimé doit être déchargé, est sensiblement perpendiculaire à ladite grande face de la bande support.

2. Cartouche selon la revendication 1, dans laquelle le corps de la cartouche est flexible et est fait d'une matière plastique choisie parmi le polyéthylène basse densité et le polypropylène.

3. Cartouche selon la revendication 1 ou 2, dans laquelle la cavité (20) de chaque récipient (16) est configurée pour correspondre sensiblement à une section droite des comprimés.

4. Cartouche selon la revendication 3, dans laquelle la cavité (20) de chaque récipient (16) possède une forme sensiblement cylindrique.

5. Cartouche selon l'une quelconque des revendications 1 à 4, dans laquelle chaque récipient (16) possède au moins une ouverture (22) communiquant avec sa cavité (20) pour permettre l'insertion d'au moins un comprimé dans la cavité ou l'éjection d'au moins un comprimé de cette cavité.

6. Cartouche selon la revendication 5, dans laquelle chaque récipient (16) possède deux ouvertures opposées, communiquant chacune avec sa cavité (20), une ouverture permettant l'accès à un dispositif d'éjection de comprimés, lequel dispositif peut s'étendre à travers cette ouverture pour éjecter le comprimé ou les comprimés à travers l'autre ouverture.

7. Cartouche selon la revendication 5 ou 6, dans laquelle au moins une ouverture est pratiquée dans la bande (14) et est couverte par une mince couche (26) se laissant facilement perforer afin de permettre l'accès à la cavité (20) des récipients respectifs à travers cette ouverture.

8. Cartouche selon l'une quelconque des revendications 5 à 7, dans laquelle chaque récipient (16) présente, à une extrémité éloignée de la bande, une ouverture qui est partiellement obturée.

9. Cartouche selon la revendication 8, dans laquelle chaque ouverture éloignée est partiellement obturée par la prévision d'un rebord.

10. cartouche selon la revendication 8, dans laquelle chaque ouverture éloignée est partiellement obturée par la prévision d'une pluralité de petites saillies (30) mutuellement espacées autour de cette ouverture.

11. Cartouche selon l'une quelconque des revendications 1 à 10, dans laquelle une pluralité de pattes (18) faisant saillie latéralement est disposée le long d'au moins un côté de la bande pour constituer un moyen par lequel le corps de la cartouche peut être indexé ou avancé pas à pas le long d'un chemin de guidage.

FIG 1

FIG 2

FIG 5

EP 0 308 269 B1

FIG 4

FIG 3